# EUROPEAN PATENT APPLICATION

(11) **EP 4 691 510 A1**
(43) Date of publication of application: **11.02.2026**
(21) Application number: 24192808.4
(22) Date of filing: 05.08.2024
(51) Int. Cl.: A61M 1/30

(54) **EXTRACOPOREAL CIRCUIT FOR SINGLE-NEEDLE AND DUAL-NEEDLE BLOOD TREATMENT**

(71) Applicant: Fresenius Medical Care Deutschland GmbH, 61352 Bad Homburg (DE)
(72) Inventor: DONARINI, Luca, 61352 Bad Homburg (DE)
(74) Representative: Lorenz Seidler Gossel Part. mbB

(57) **Abstract**

The present invention pertains to a tube set configured to be suitable both for single-needle and dual-needle hemodialysis. The tube set comprises an extracorporeal circuit comprising an arterial line configured to convey blood from a patient to a blood pump of a blood treatment device, and a venous line configured to convey blood from the blood pump to the patient, wherein the arterial line and the venous line each comprise a line connector configured to removably fluidically couple the arterial line and the venous line to an expansion chamber that is removably cou-pleable to the extracorporeal circuit. The removable expansion chamber allows the same tube set to be used for single-needle and dual-needle (conventional) hemodialysis.

## Description

The present invention pertains to an extracorporeal circuit for single-needle and dual-needle blood treatment comprising a removable expansion chamber.

The present invention also pertains to a tube set comprising such an extracorporeal ciruit and such a removable expansion chamber and a blood treatment device comprising such a tube set.

In modern extracorporeal blood treatments, such as hemodialysis, disposable tube sets are used. Often, specific treatments require specific tube sets comprising the fluid lines, containers and connectors etc. required for the specific treatment.

This has the disadvantage that hospitals need to stock multiple different tube sets to be able to offer a variety of blood treatments, such as for example single-needle hemodialysis and conventional, dual-needle, hemodialysis.

It is an object of the present invention to alleviate or even completely abolish the disadvantages of the prior art. In particular, it is an object of the present invention to provide a tube set suitable for both single-needle hemodialysis and dual-needle hemodialysis.

In single-needle hemodialysis, a single patient access structure, e.g. a needle or catheter is used, to alternatively remove blood from the patient and return it to the patient. In dual-needle hemodialysis (the most common hemodialysis), the arterial line and the venous line each have a designated patient access structure, e.g. a needle or catheter, to remove blood from the patient and return it to the patient.

The object of the present invention is achieved by the subject-matter of the independent claims. The dependent claims recite embodiments of the invention.

A first aspect of the invention pertains to an extracoporeal circuit for single-needle and dual-needle blood treatment, comprising:
an arterial line configured to convey blood from a patient to a blood pump of a blood treatment device, and
a venous line configured to convey blood from the blood pump via the dialyzer to the patient, wherein the arterial line and the venous line each comprise a line connector configured to removably fluidically couple the arterial line and the venous line to an expansion chamber that is removably coupleable to the extracorporeal circuit.

The expansion chamber preferably is a container for storing fluid, such as blood. The expansion chamber can be a flexible bag or a rigid container, in both cases preferably made of polymer material.

The line connector of the arterial line can be different from the line connector of the venous line to prevent erroneous connections. This preferably ensures that each line connector can only be connected to the correct and corresponding chamber connector of the expansion chamber, so that the expansion chamber can be coupled to the extracorporeal circuit only in the desired way.

The line connector of the arterial line and / or the venous line can be arranged on a branch line of the arterial line or venous line. The branch line preferably branches off of the arterial line or venous line immediately before or after a a section of the circuit that interacts with the blood pump, for example a pump loop occluded by the pump in case of a roller pump.

If the extracorporeal circuit is to be used for dual-needle, standard hemodialysis treatment, the expansion chamber is preferable not coupled to the circuit. For this application, the branch lines and / or line connectors need to be fluidically closed to prevent the leaking of fluid from the circuit.

This closing function can be achieved e.g. by a closing element present on a blood treatment device. In other words, the branch line of the arterial line and / or venous line can be configured to be opened and closed by a closing element, preferably a valve or clamp, preferably of a blood treatment device.

Of course, the closing element does not have to be part of a blood treatment device, but can also be e.g. a manual clamp present on the branch line. If the closing element is part of a blood treatment device, this has the advantage that the closing elemnt can be automatically controlled e.g. by a control unit of the device.

Alternatively or additionally, the line connector of the arterial line and / or the venous line can comprise a sealing element, preferably a cap or valve, in particular check valve, or piercable septum, configured to prevent the leaking of fluid from the connector.

The sealing element can be configured to be opened by a corresponding chamber connector upon connection of the expansion chamber to the circuit.

The arterial line and the venous line can be connectable or connected to a common patient access structure, such as a catheter, to be suitable and / or configured for single-needle treatment or the arterial line and the venous line are each connectable or connected to a separate patient access structure, such as a catheter, to be suitable and / or configured for dual-needle treatment.

Another aspect of the present invention pertains to a removable expansion chamber to be used preferably with an extracorporeal circuit according to the present invention.

According to the invention, the expansion chamber and circuit are separate entities that can, for example, be purchased separately by a hospital. This has the advantage that not the same numbers of circuits and expansion chambers have to be purchased.

If a hospital predominantly offers dual-needle hemodialysis but wishes to also offer single-needle dialysis to a lesser degree, it may be advantageous to purchase a smaller number of expansion chambers than circuits so that upon demand a circuit according to the present invention can be equipped for single-needle use without the need of stocking a large number of specialised tube sets.

An expansion chamber according to the present invention is preferably configured to be used with an extracorporeal circuit according to the present invention and comprises:
an inlet line configured to convey fluid into the expansion chamber, and
an outlet line configured to convey fluid out of the expansion chamber, wherein the inlet line and the outlet line each comprise a chamber connector configured to interact with the line connectors of the extracorporeal circuit to removably fluidically couple the expansion chamber to the extracorporeal circuit.

The chamber connector of the inlet line can be different from the chamber connector of the outlet line to prevent erroneous connections. For example, each chamber connector of the expansion chamber can be geometrically configured to be able to connect only to the corresponding line connector of the circuit.

The chamber connector of the inlet line and / or the outlet line can comprise an opening structure configured to fluidically open the line connector, preferably its sealing element, of the arterial line and / or venous line upon connection of the chamber connector to the corresponding line connector.

Of course, the opposite case is also possible, wherein the opening structure is present on the line connector side and the sealing structure is present on the chmber connector side. The inlet line and / or the outlet line can also comprise a check valve to prevent the undesired leakage of fluid.

The expansion chamber can further comprise a monitoring element configured to monitor a filling level of the expansion chamber, wherein the monitoring element preferably is a pressure transducer or a weighing device.

For example, the expansion chamber can be positioned on top of the monitoring element that measures a weight or pressure exerted by the expansion chamber. The monitoring element can also be e.g. arranged at a hook at that the expansion chamber is hung. The monitoring element can also comprise e.g. a strain transducer that measures the strain exerted by the expansion chamber e.g. on a support structure.

In principle, the monitoring element can be configured to measure any parameter indicative of the filling level of the expansion chamber.

A further aspect of the present invention pertains to a tube set for blood treatment, comprising an extracorporeal circuit according to the present invention and an expansion chamber according to the present invention, wherein the expansion chamber is coupled or coupleable (or connectable or connected) to the extracorporeal circuit.

In the tube set, for example, the line connector of the arterial line is removably coupled to the chamber connector of the outlet line and / or the line connector of the venous line is removably coupled to the chamber connector of the inlet line, so that the expansion chamber is removably fluidically coupled to the extracorporeal circuit.

The line connector of the arterial line can be configured to be exclusively coupleable to the chamber connector of the outlet line and / or the line connector of the venous line can be configured to be exclusively coupleable to the chamber connector of the inlet line. This ensures correct connection of the expansion chamber to the circuit.

Another aspect of the present invention pertains to a system comprising a blood treatment device and a tube set according to the present invention.

The blood treatment device preferably comprises a control unit configured to monitor a filling level of the expansion chamber, wherein the control unit preferably monitors the filling level of the expansion chamber by determining a volume pumped by the blood pump, preferably based on a number of pump strokes and a stroke volume of the blood pump.

Alternatively or additionally, the control unit of the blood treatment device can access the measurement values of the monitoring element of the expansion chamber.

Preferably, the control unit is further configured to operate the system to perform a single-needle or dual-needle blood treatment, preferably haemodialysis. Preferably, the blood treatment device is a dialysis, in particular hemodialysis, device.

Preferably, the blood treatment device comprises only one blood pump.

It is to be noted that if in the present disclosure a singular article such as "a" or "the" is used, the disclosure is not limited to this singular form, but the element is question can also be present in plural. Thus, thses articles are to be understood as "at least one".

Furthermore, it is to be noted that the present disclosure is not limited to the embodiments explicitly recited therein, but all features disclosed herein can be combined with each other in other combinations or claimed in isolation, as desired.

Further aspects, effects and features of the present invention become apparent from the following description of embodiments of the invention under reference to the figures in which like reference signs denote like elements.
Fig. 1 shows a tube set according to the present invention, wherein the expansion chamber is coupled to the circuit;
Fig. 2 shows the tube set of Fig. 1 with the expansion chamber removed from the circuit;
Fig. 3 illustrates the use of the circuit of Fig. 2 for dual-needle hemodialysis;
Fig. 4 illustrates the use of the circuit of Fig. 2 for single-needle hemodialysis and shows a filling phase of the expansion chamber, and
Fig. 5 illustrates the use of the circuit of Fig. 2 for single-needle hemodialysis and shows a draining phase of the expansion chamber.

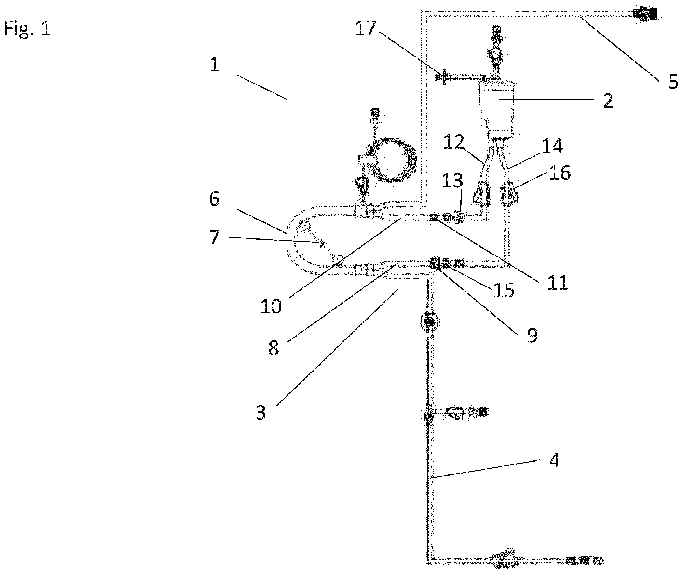

Fig. 1 shows a tube set 1 according to an embodiment of the present invention, wherein the expansion chamber 2 is coupled to the circuit 3.

The circuit 3 comprises an arterial line 4, a venous line 5 with a connector configured to connect the line to a dialyzer and a pump loop section 6 configured to interact with a blood pump (roller pump) 7.

From the arterial line 4, a branch line 8 branches off just next to the pump loop section 6. The branch line 8 comprises a line connector 9.

From the venous line 5, a branch line 10 branches off just next to the pump loop section 6. The branch line 10 comprises a line connector 11.

The expansion chamber 2 comprises an inlet line 12 that comprises a chamber connector 13. The chamber connector 13 is removably connected to the line connector 11.

The expansion chamber 2 further comprises an outlet line 14 that comprises a chamber connector 15. The chamber connector 15 is removably connected to the line connector 9.

The inlet line 12 and the outlet line 14 each comprise a closing element in the form of a clamp 16, preferably a manual or automatic clamp.

The expansion chamber is further equipped with a pressure transducer 17 to monitor the filling level of the expansion chamber 2.

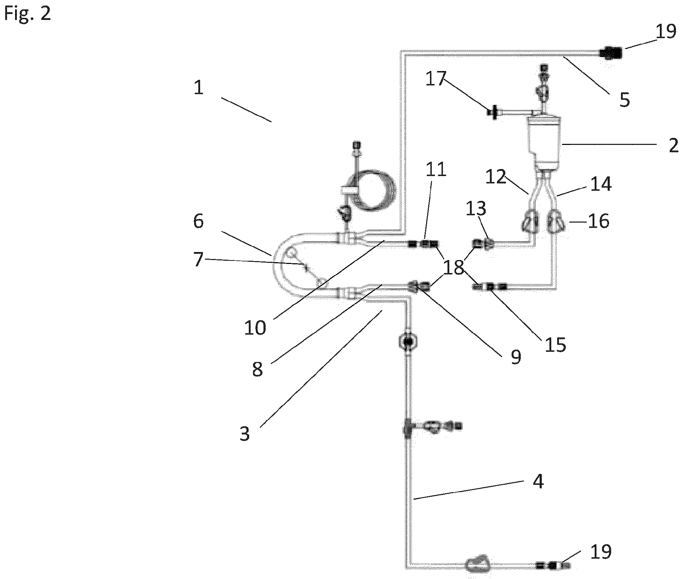

Fig. 2 shows the tube set 1 of Fig. 1 with the expansion chamber 2 removed from the circuit 3.

In this configuration, the line connectors 9 and 11 and the chamber connectors 13 and 15 are closed or sealed with caps 18.

The arterial line 4 comprises a connector 19 configured to link the lines to a patient access structure, e.g. to a common catheter for single-needle treatment or to a separate catheter for each line for dual-needle treatment.

The venous line 5 comprises a connector 19 configured to connect the line to a dialyzer and a pump loop section 6 configured to interact with a blood pump (roller pump) 7.

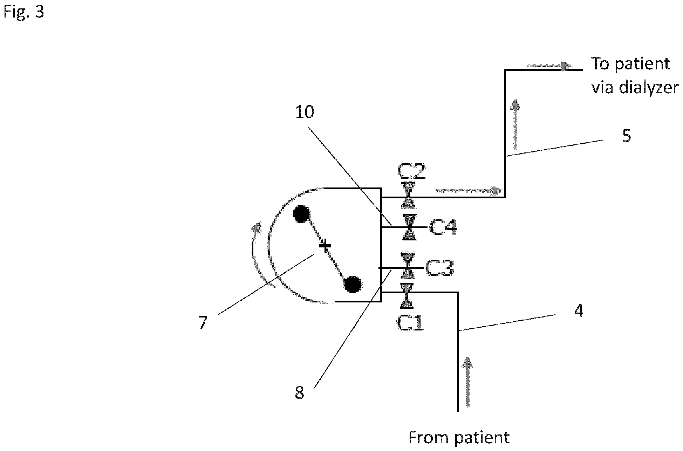

Fig. 3 illustrates the use of the circuit 3 of Fig. 2 for dual-needle hemodialysis. For this application, no expansion chamber is coupled to the circuit 3 and the branch lines 8 and 10 are closed.

In the example of Fig. 3, the branch lines 8 and 10 are closed off by automatic clamps C3, C4 (e.g. electromagnetic clamps) of a blood treatment device, such as a hemodialysis device.

The automatic clamps C1 and C2 (e.g. electromagnetic clamps) of the blood treatment device are open during the operation as shown in Fig. 3.

Thus, blood flows as indicated by the arrows through the arterial line 4, the open clamp C1, the blood pump 7, the open clamp C2 and into the venous line 5 to the dialyzer.

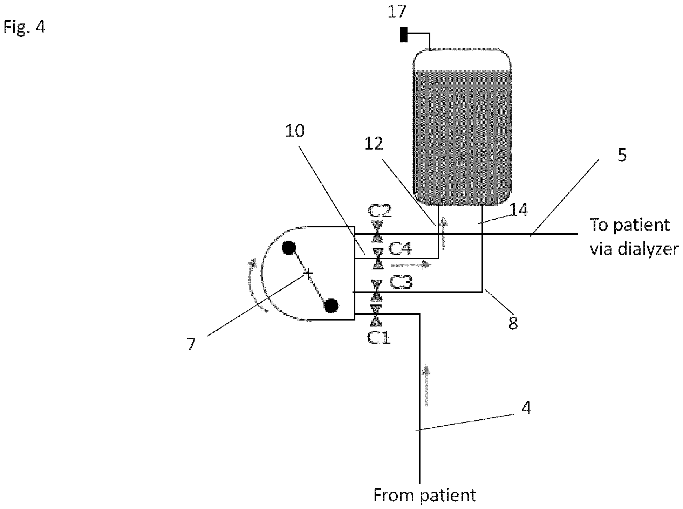

Fig. 4 illustrates the use of the circuit 3 of Fig. 2 for single-needle hemodialysis and shows a filling phase of the expansion chamber 2.

For this application, an expansion chamber 2 is coupled to the circuit 3 and the branch lines 8 and 10 are opened and closed via the clamps C3 and C4 to fill and drain the expansion chamber 2.

As Fig. 4 shows a filling phase of the expansion chamber 2, clamps C1 and C4 are open and clamps C2 and C3 are closed, so blood flows through the arterial line 4, the open clamp C1, the blood pump 7, the open clamp C4, the branch line 10, the inlet line 12 and into the expansion chamber 2.

The filling level / volume of the expansion chamber is monitored by a pressure transducer 17. The blood is temporarily stored in the expansion chamber 2.

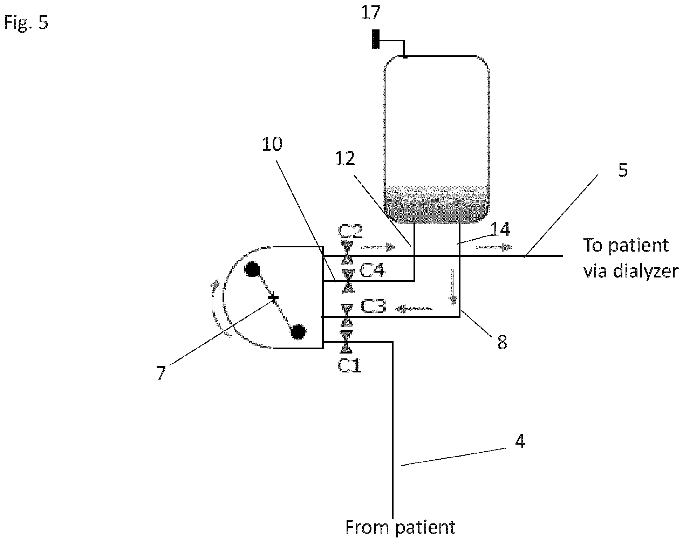

Fig. 5 illustrates the use of the circuit 3 of Fig. 2 for single-needle hemodialysis and shows a draining phase of the expansion chamber 2.

As Fig. 5 shows a draining phase of the expansion chamber 2, clamps C3 and C2 are open and clamps C1 and C4 are closed, so blood flows through the outlet line 14, the branch line 8, the open clamp C3, the blood pump 7, the open clamp C2 and into the venous line 5 to the dialyzer.

## Claims

1. Extracoporeal circuit for single-needle and dual-needle blood treatment, comprising:
an arterial line configured to convey blood from a patient to a blood pump of a blood treatment device, and
a venous line configured to convey blood from the blood pump to the patient, wherein the arterial line and the venous line each comprise a line connector configured to removably fluidically couple the arterial line and / or the venous line to an expansion chamber that is removably coupleable to the extracorporeal circuit.

2. Extracorporeal circuit according to claim 1, wherein the line connector of the arterial line is different from the line connector of the venous line to prevent erroneous connections.

3. Extracorporeal circuit according to claim 1 or 2, wherein the line connector of the arterial line and / or the venous line is arranged on a branch line of the arterial line or venous line.

4. Extracorporeal circuit according to claim 3, wherein the branch line of the arterial line and / or venous line is configured to be opened and closed by a closing element, preferably a valve or clamp, preferably of a blood treatment device.

5. Extracorporeal circuit according to one of the preceding claims, wherein the line connector of the arterial line and / or the venous line comprises a sealing element, preferably a cap or valve, in particular a check valve, or piercable septum, configured to prevent the leaking of fluid from the connector.

6. Extracorporeal circuit according to one of the preceding claims, wherein the arterial line and the venous line are connected to a common patient access structure, such as a catheter, to be suitable and / or configured for single-needle treatment, or wherein the arterial line and the venous line are each connected to a separate patient access structure, such as a catheter, to be suitable and / or configured for dual-needle treatment.

7. Expansion chamber, configured to be used with an extracorporeal circuit according to one of the preceding claims, comprising
an inlet line configured to convey fluid into the expansion chamber, and
an outlet line configured to convey fluid out of the expansion chamber,
wherein the inlet line and the outlet line each comprise a chamber connector configured to interact with the line connectors of the extracorporeal circuit to removably couple the expansion chamber to the extracorporeal circuit.

8. Expansion chamber according to claim 7, wherein the chamber connector of the inlet line is different from the chamber connector of the outlet line to prevent erroneous connections.

9. Expansion chamber according to claim 7 or 8, wherein the chamber connector of the inlet line and / or the outlet line comprises an opening structure configured to fluidically open the line connector, preferably its sealing element, of the arterial line and / or venous line upon connection of the chamber connector to the corresponding line connector.

10. Expansion chamber according to one of claims 7 to 9, further comprising a monitoring element configured to monitor a filling level of the expansion chamber, wherein the monitoring element preferably is a pressure transducer or a weighing device.

11. Tube set for blood treatment, comprising:
an extracorporeal circuit according to one of claims 1 to 6 and an expansion chamber according to one of claims 7 to 10, wherein the expansion chamber is coupled or coupleable to the extracorporeal circuit.

12. Tube set for blood treatment according to claim 11, wherein the line connector of the arterial line is removably coupled to the chamber connector of the outlet line and / or the line connector of the venous line is removably coupled to the chamber connector of the inlet line, so that the expansion chamber is removably fluidically coupled to the extracorporeal circuit.

13. Tube set for blood treatment according to claim 11 or 12, wherein the line connector of the arterial line is configured to be exclusively coupleable to the chamber connector of the outlet line and / or the line connector of the venous line is configured to be exclusively coupleable to the chamber connector of the inlet line.

14. System comprising a blood treatment device and a tube set of one of claims 11 to 13, wherein the blood treatment device comprises a control unit configured to monitor a filling level of the expansion chamber, wherein the control unit preferably monitors the filling level of the expansion chamber by determining a volume pumped by the blood pump, prefrerably based on a number of pump strokes and a stroke volume of the blood pump.

15. System according to claim 14, wherein the control unit is further configured to operate the system to perform a single-needle or dual-needle blood treatment, preferably haemodialysis.
